# EUROPÄISCHE PATENTSCHRIFT

(11) **EP 2 621 423 B1**
(45) Veröffentlichungstag und Bekanntmachung des Hinweises auf die Patenterteilung: **05.11.2014**
(21) Anmeldenummer: 11805750.4
(22) Anmeldetag: 24.09.2011
(51) Int. Cl.: A61F 9/007

(54) **STEUERUNGSVORRICHTUNG FÜR EIN OPHTHALMOCHIRURGISCHES SYSTEM**
CONTROL DEVICE FOR AN OPHTHALMIC SURGICAL SYSTEM
DISPOSITIF DE COMMANDE D'UN SYSTÈME DE CHIRURGIE OPHTALMOLOGIQUE

(30) Priorität: 30.09.2010 DE 102010047012
(43) Veröffentlichungstag der Anmeldung: 07.08.2013
(73) Patentinhaber: Carl Zeiss Meditec AG, 07745 Jena (DE)
(72) Erfinder: KUEBLER, Christoph, 73447 Oberkochen (DE); WEHNER, Wolfram, 90402 Nürnberg (DE); REIN, Karlheinz, 73430 Aalen (DE)
(74) Vertreter: Carl Zeiss AG - Patentabteilung
(86) Internationale Anmeldenummer: PCT/DE2011/001775
(87) Internationale Veröffentlichungsnummer: WO 2012/041288

(56) Entgegenhaltungen:
- WO-A2-2005/092023
- DE-A1-102008 046 687
- US-B1- 6 544 254

## Beschreibung

Die Erfindung betrifft eine Steuerungsvorrichtung für ein ophthalmochirurgisches System in Abhängigkeit von einer Okklusion sowie ein ophthalmochirurgisches System mit einer solchen Steuerungsvorrichtung.

Zur Behandlung einer Augenlinsentrübung, welche in der Medizin als Grauer Star bezeichnet wird, gibt es mehrere chirurgische Techniken. Die am weitesten verbreitete Technik ist die Phakoemulsifikation, bei der eine dünne Nadel in die erkrankte Linse eingeführt und mit Ultraschall zu Schwingungen angeregt wird. Die vibrierende Nadel emulsifiziert in ihrer nächsten Umgebung die Linse derart, dass die entstehenden Linsenpartikel durch eine Leitung mittels einer Pumpe abgesaugt werden können. Dabei wird ein Spülfluid (Irrigationsfluid) zugeführt, wobei das Absaugen der Partikel und des Fluides durch eine Aspirationsleitung erfolgt. Ist die Linse vollständig emulsifiziert und entfernt worden, kann in den leeren Kapselsack eine neue künstliche Linse eingesetzt werden, so dass ein derart behandelter Patient wieder ein gutes Sehvermögen erreichen kann.

Beim Emulsifizieren kann ein Partikel so an die Ansaugöffnung der Aspirationsleitung in einer schwingenden Nadel herangezogen werden, dass die Aspirationsleitung verstopft. Ein solcher Zustand wird als Okklusion bezeichnet. In diesem Fall können kein Fluid und keine anderen zertrümmerten Linsenpartikel mehr in die Aspirationsleitung gelangen. Wenn eine Absaugpumpe unverändert weiter betrieben wird, baut sich in der Aspirationsleitung ein starker Unterdruck auf. Der Unterdruck reicht in der Regel nicht aus, um die die Nadelspitze verstopfenden Partikel durch die Aspirationsleitung hindurch zu saugen. Eine Möglichkeit, die Okklusion zu beenden, besteht darin, die Nadel mit einer höheren Ultraschallenergie zu betreiben, so dass das Partikel an der Nadelspitze in kleinere Partikel zerbricht und die Okklusion beendet wird. Der höhere Energieeintrag zum Zertrümmern von Linsenpartikeln führt jedoch zu dem unerwünschten Effekt, dass die Nadel auch das umliegende Gewebe stark erwärmt. Da die Nadel bei der Operation durch die Hornhaut gestochen wird, erwärmt sich dadurch die Hornhaut, so dass diese bei zu langem und hohem Energieeintrag in die Augenlinse mit beschädigt wird (Cornea Burn). Eine solche Verletzung eines Patientenauges kann vermieden werden, wenn für eine längere Zeit die erforderliche Ultraschallenergie zum Zertrümmern von Partikeln auf einen niedrigen Wert gesetzt wird. Dies verlängert aber die Operationsdauer.

Es ist eine Aufgabe der Erfindung, eine Steuerungsvorrichtung für ein ophthalmochirurgisches System vorzuschlagen, mit dem in kurzer Zeit eine Phakoemulsifikation der gesamten Augenlinse durchführbar ist, wobei das Risiko für eine Beschädigung des Patientenauges gering gehalten wird. Ferner ist es eine Aufgabe, ein ophthalmochirurgisches System mit einer derartigen Steuerungsvorrichtung zu schaffen.

Die Aufgabe wird für die Steuerungsvorrichtung durch den Gegenstand des unabhängigen Patentanspruchs 1 gelöst. Vorteilhafte Weiterbildungen der Erfindung sind Gegenstand der Unteransprüche. Die Aufgabe für das ophthalmochirurgische System wird durch den Gegenstand des unabhängigen Patentanspruchs 6 gelöst.

Die erfindungsgemäße Steuerungsvorrichtung für ein ophthalmochirurgisches System weist auf:
- eine Durchflussbestimmungsvorrichtung, mit welcher sich ein Istwert eines Fluiddurchflusses in einer Aspirationsleitung bestimmen lässt, wobei die Aspirationsleitung mit einem Handstück zur Phakoemulsifikation einer Augenlinse gekoppelt ist,
- eine Okklusionsbestimmungsvorrichtung, mit welcher sich bestimmen lässt, ob an einer Ansaugöffnung der Aspirationsleitung eine Okklusion durch ein Partikel der Augenlinse vorliegt,
- eine Auswerteeinheit, welche geeignet ist, in Abhängigkeit von dem bestimmten Istwert des Fluiddurchflusses in der Aspirationsleitung eine Härte des Partikels der Augenlinse zu ermitteln, wenn von der Okklusionsbestimmungsvorrichtung bestimmt worden ist, dass eine Okklusion vorliegt, und in Abhängigkeit von der Härte einen ersten Betrag einer Ultraschallenergie zu bestimmen, welche einem Handstück mittels einer Energiequelle zuführbar ist, und
- eine Steuereinheit, mit welcher sich die Energiequelle so ansteuern lässt, dass sie während der Okklusion den ermittelten ersten Betrag der Ultraschallenergie abgibt.

Die Erfindung setzt somit bei dem Gedanken an, in Abhängigkeit von einem bestimmten Istwert des Fluiddurchflusses in der Aspirationsleitung eine Härte der Augenlinse zu ermitteln. Die Erfinder haben festgestellt, dass auch mit einer relativ niedrigen Ultraschallenergie eine ausreichend kleine Größe von Linsenpartikeln erzeugt und eine Okklusion aufgebrochen werden kann, wenn die Härte der Linse relativ niedrig ist. Ein höherer Ultraschallenergiebetrag ist jedoch erforderlich, wenn die Linsenpartikel eine relativ hohe Härte besitzen. Wenn somit eine Aussage über eine Härte der zu emulsifizierenden Linsenpartikel getroffen werden kann, lässt sich anhand eines solchen Härtewertes ein maximal zur Verfügung gestellter Betrag einer Ultraschallenergie einstellen. Dadurch wird vermieden, dass zum Beispiel bei einem weichen Linsenpartikel zu viel Energie zugeführt wird, so dass das Risiko einer Verbrennung der Hornhaut reduziert wird. Wenn die gesamte Augenlinse nur aus relativ weichem Material besteht, genügt es, mit einem relativ niedrigen Betrag an Ultraschallenergie die Nadel zu betreiben. Falls der Linsenwerkstoff jedoch teilweise aus einem harten und teilweise aus einem weichen Bereich besteht, kann durch die jeweilige Bestimmung der Härte der zu zertrümmernden Linsenpartikel die Ultraschallenergie zugeführt werden, die gerade erforderlich ist, um die Partikel zu zertrümmern. Eine derartige Steuerung einer Ultraschallenergie ist wesentlich effizienter und schneller als zum Beispiel die manuelle Steuerung durch ein Fußpedal, welches vom Chirurgen betätigt werden muss.

Durch die erfindungsgemäße Steuerungsvorrichtung wird auch sichergestellt, dass nur der minimal erforderliche Energieeintrag zum Zertrümmern der jeweiligen Partikel zugeführt wird. Bei weichen Partikeln wird wenig Energie zugeführt., bei harten Partikeln wird mehr Energie zugeführt. Dies verringert die Wahrscheinlichkeit, dass gesundes Gewebe in der Umgebung der Nadel, wie zum Beispiel die Hornhaut, durch Überhitzung verletzt wird. Zusätzlich erfolgt die Energiezufuhr nur in dem Fall, wenn eine Okklusion erkannt worden ist. Falls die Partikel so klein sind, dass sie problemlos durch die Aspirationsleitung gesaugt werden können, wird auch keine Ultraschallenergie zugeführt. Dies verringert nochmals den Betrag an zugeführter Energie und verringert die Wahrscheinlichkeit einer Verletzung des Patientenauges. Trotzdem ist nur ein geringer Zeitaufwand für die Zertrümmerung der Linsenpartikel erforderlich. Somit wird die gestellte Aufgabe durch die erfindungsgemäße Steuerungsvorrichtung gelöst.

In einer bevorzugten Ausführungsform ist die Steuereinheit geeignet, die Energiequelle so anzusteuern, dass sie einen zweiten Betrag einer Ultraschallenergie abgibt, wenn von der Okklusionsbestimmungsvorrichtung bestimmt worden ist, dass keine Okklusion vorliegt, wobei der zweite Betrag niedriger als der erste Betrag ist. Wenn keine Okklusion vorliegt, sind auch keine die Nadel verstopfenden Partikel zu zertrümmern. Trotzdem kann es sinnvoll sein, auch ohne Vorliegen einer Okklusion die Nadel des Phakohandstückes mit einem niedrigen Betrag einer Ultraschallenergie anzusteuern. Dies kann die Absaugung unterstützen und den Transport von kleinen Partikeln gemäß dem Prinzip einer Schwingrinne nachbilden. Wenn der zweite Betrag einer Ultraschallenergie niedriger als der erste Betrag der Ultraschallenergie bei einer Okklusion ist, kann trotzdem die Gefahr einer Beschädigung des Patientenauges vermieden werden.

Vorzugsweise weist die Okklusionsbestimmungsvorrichtung eine Messvorrichtung zum Messen des von einer Aspirationspumpe aufgenommenen Stroms oder einer Pumpenleistung oder des Fluiddurchflusses in der Aspirationsleitung auf. Wenn das Partikel die Nadel verstopft, kann die Aspirationspumpe so angetrieben werden, dass diese versucht, durch einen höheren Unterdruck das Partikel noch durch die Aspirationsleitung zu saugen. In diesem Fall nimmt der von der Pumpe gezogene Strom zu, so dass die Stromstärke der Pumpe ein Parameter für eine Okklusion bildet. Selbstverständlich kann auch das Produkt aus dem gezogenen Strom mit der anliegenden Pumpenspannung, also die Pumpenleistung, für eine Detektion einer Okklusion benutzt werden. Alternativ kann die Okklusionsbestimmungsvorrichtung eine Okklusion auch mittels eines Fluiddurchflusses in der Aspirationsleitung erkennen. Bei einer Okklusion nimmt der Fluiddurchfluss deutlich ab.

Gemäß einer Weiterbildung der Erfindung ist die Auswerteeinheit geeignet, die Härte des Linsenpartikels in Abhängigkeit von einem Quotienten aus dem Istwert des Fluiddurchflusses zu einem vorbestimmten Sollwert des Fluiddurchflusses zu ermitteln. Der Sollwert des Fluiddurchflusses kann vom Chirurgen vor Beginn einer Behandlung bestimmt werden. Ein solcher Sollwert kann abhängen von verwendeten Pulsmustern bei einer durch Pulse zugeführten Ultraschallenergie, oder von mechanischen Abmessungen der Nadel des Phakohandstückes.

Vorzugsweise ist die Auswerteeinheit geeignet, während einer vorbestimmten Zeitdauer ein Integral des Quotienten aus dem Istwert des Fluiddurchflusses zu dem Sollwert des Fluiddurchflusses über die Zeit zu bestimmen. Damit können Beträge eines derartigen Quotienten über eine vorbestimmte Zeit aufaddiert werden. Kurzzeitige Zitterbewegungen des Partikels und damit einhergehende starke Schwankungen des Fluiddurchflusses führen bei einer solchen Integration über eine längere Zeitdauer nicht mehr zu einem hektisches Umschalten von einem ersten Betrag einer Ultraschallenergie zu einem zweiten Betrag einer Ultraschallenergie. Ferner kann durch die Integration der momentanen Beträge eine Härte des zu zertrümmernden Partikels genauer und zuverlässiger bestimmt werden.

Weitere Vorteile und Merkmale der Erfindung werden mit Bezug auf die nachfolgenden Zeichnungen erklärt, in welchen zeigen:
- Figur 1: eine schematische Darstellung einer ersten Ausführungsform eines ophthalmochirurgischen Systems mit einer erfindungsgemäßen Steuerungsvorrichtung;
- Figur 2: eine schematische Darstellung eines harten und eines weichen Partikels am Rand einer Ansaugöffnung einer Nadel eines Phakohandstückes; und
- Figur 3: ein Diagramm mit Kurvenverläufen in Abhängigkeit von der Zeit bezüglich eines Quotienten aus einem Istwert eines Fluiddurchflusses und einem Sollwert eines Fluiddurchflusses, einer Okklusion, einer Härte eines zu zertrümmernden Partikels und einer Ultraschallenergie.

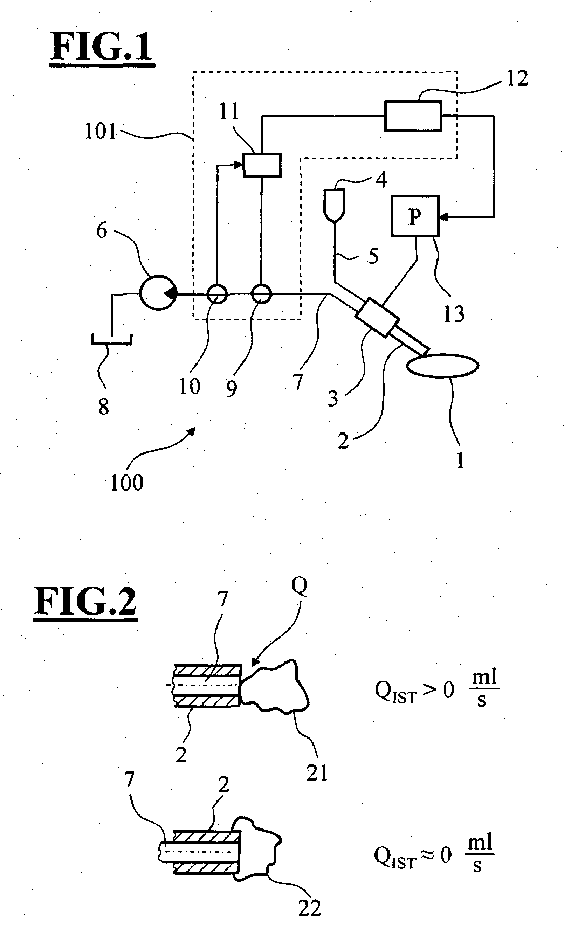

Figur 1 zeigt eine schematische Darstellung einer Ausführungsform eines ophthalmochirurgischen Systems 100 mit einer Steuerungsvorrichtung 101. Eine zu behandelnde Augenlinse 1 wird während einer Phakoemulsifikation von einer Nadel 2 eines Handstückes 3 bearbeitet. Von einem Irrigationsfluidbehälter 4 fließt Irrigationsfluid durch eine Irrigationsleitung 5 zum Handstück 3 und dort zu einer Nadel 2, deren Spitze die Augenlinse 1 berührt. Das zugeführte Irrigationsfluid und die zertrümmerten Linsenpartikel werden über eine Aspirationsleitung 7, welche durch die Nadel 2 und das Handstück 3 zu einer Aspirationspumpe 6 führt, abgesaugt. Das Fluid und die Partikel werden dann in einem Aspirationsbehälter 8 aufgefangen. Mit der Aspirationsleitung 7 ist eine Durchflussbestimmungsvorrichtung 9 gekoppelt, mit welcher ein Istwert eines Fluiddurchflusses bestimmbar ist. Ferner ist mit der Aspirationsleitung 7 eine Okklusionsbestimmungsvorrichtung 10 vorgesehen, mit welcher sich bestimmen lässt, ob an der Nadel 2 des Handstückes 3 eine Okklusion durch ein Partikel der Augenlinse 1 vorliegt. Die Okklusionsbestimmungsvorrichtung 10 kann eine Messvorrichtung zum Bestimmen eines Fluiddurchflusses in der Aspirationsleitung 7 aufweisen.

Wenn von der Durchflussbestimmungsvorrichtung 9 ein Istwert eines Fluiddurchflusses in der Aspirationsleitung 7 bestimmt wird, kann dieser Istwert zu einer Auswerteeinheit 11 geleitet werden, welche in Abhängigkeit von dem bestimmten Istwert eine Härte des zu zertrümmernden Partikels der Augenlinse ermittelt. Wenn der Fluiddurchfluss nur gering abnimmt, so dass ein kleiner Teil der Aspirationsleitung 7 an der Spitze der Nadel 2 noch frei ist und Fluid bzw. kleinste Partikel hindurch gesogen werden können, liegt nur eine relativ schwache Okklusion vor, welche von der Okklusionsbestimmungsvorrichtung 10 aber erkannt wird. Die Erfinder haben beobachtet, dass eine solche Situation vorliegt, wenn ein zu zertrümmerndes Partikel relativ hart ist. Wird von der Durchflussbestimmungsvorrichtung jedoch ermittelt, dass der Istwert nur noch relativ niedrig ist, so liegt nach der Erfahrung der Erfinder ein relativ weiches Partikel vor.

In Abhängigkeit von der Härte wird von der Auswerteeinheit 11 ein erster Betrag einer Ultraschallenergie bestimmt, mit welcher das Partikel vor der Nadel 2 beaufschlagt werden soll. Diese Information wird einer Steuereinheit 12 zugeführt, mit welcher sich eine Energiequelle 13 so ansteuern lässt, dass sie während der Okklusion den ermittelten ersten Betrag der Ultraschallenergie an ein Handstück 3 abgibt.

Figur 2 erläutert die Situation an der Spitze einer Nadel, wenn ein hartes Partikel 21 oder ein weiches Partikel 22 vorliegt. Bei einem harten Partikel 21 verbleibt gemäß der Beobachtung der Erfinder immer ein kleiner Bereich der Aspirationsleitung 7 an der Spitze der Nadel 2, durch welcher ein Fluid oder kleinste Partikel noch hindurch gesogen werden können. Der Durchfluss Q_{IST} ist größer als null. Bei einem weichen Partikel 22 hingegen wird die gesamte Ansaugöffnung der Aspirationsleitung 7 verstopft. Ein Fluiddurchfluss kommt bei einem weichen Partikel somit fast zum Erliegen. Durch den Ansaugdruck in der Aspirationsleitung wird ein weiches Partikel vollständig an eine Spitze einer Nadel herangesogen. Bei einem harten Partikel fehlt diese Elastizität, so dass immer ein kleiner Bereich übrig bleibt, durch den noch Fluid angesogen werden kann. Der Betrag eines solchen Fluiddurchflusses durch die Aspirationsleitung bzw. der Quotient aus dem Istwert eines Fluiddurchflusses durch einen Sollwert eines Fluiddurchflusses kann damit eine Grundlage bilden, um daraus eine Härte eines zu zertrümmernden Partikels zu ermitteln und daraufhin die erforderliche Energie festzulegen, um dieses Partikel zu zertrümmern.

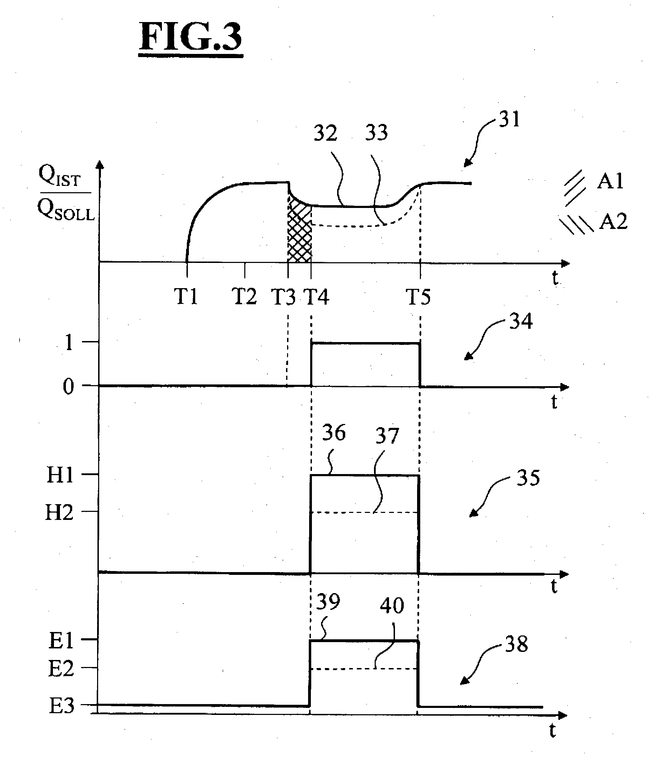

Figur 3 zeigt für eine erste Ausführungsform der erfindungsgemäßen Steuerungsvorrichtung mehrere Kurvenverläufe in Abhängigkeit von der Zeit. Das oberste Diagramm 31 zeigt einen Kurvenverlauf eines Quotienten aus einem Istwert eines Fluiddurchflusses Q_{IST} durch einen Sollwert eines Fluiddurchflusses Q_{SOLL} in Abhängigkeit von der Zeit t. Zum Zeitpunkt T1 wird die Aspirationspumpe 6 eingeschaltet, so dass sich bis zum Zeitpunkt T2 ein stationärer Wert für den Quotienten aus dem Istwert des Fluiddurchflusses Q_{IST} und dem Sollwert des Fluiddurchflusses Q_{SOLL} einstellt. Wenn sich ein Partikel 21 oder 22 an der Nadelspitze anlegt, verringert sich der Quotient aus dem Istwert des Fluiddurchflusses Q_{IST} und dem Sollwert des Fluiddurchflusses Q_{SOLL}. Gemäß der ersten Ausführungsform kann der Zeitpunkt, an dem eine solche Verringerung des Quotienten beginnt, als T3 definiert werden. Gemäß einer zweiten Ausführungsform kann der Zeitpunkt, an dem der Quotient aus Q_{IST} zu Q_{SOLL} kleiner als ein vorbestimmter Schwellwert ist, als T3 definiert werden.

Der Kurvenverlauf 32 zeigt an, dass der Quotient aus Q_{IST} zu Q_{SOLL} vom Zeitpunkt T3 bis zum Zeitpunkt T4 absinkt. Die Zeitdauer zwischen T3 und T4 ist entweder fest voreingestellt oder kann vom Benutzer vor Beginn einer ophthalmochirurgischen Behandlung bestimmt werden. Wenn zum Zeitpunkt T4 der Wert des Quotienten aus dem Istwert des Fluiddurchflusses Q_{IST} und dem Sollwert des Fluiddurchflusses Q_{SOLL} kleiner als ein vorbestimmter Schwellwert ist, ermittelt die Okklusionsbestimmungsvorrichtung 10 daraus, dass eine Okklusion vorliegt, so dass ein Signal von 0 auf 1 springt, siehe Diagramm 34. Tatsächlich kann eine Okklusion bereits ab dem Zeitpunkt T3 vorliegen, jedoch wird durch die erfindungsgemäße Steuerungsvorrichtung erst zum Zeitpunkt T4 ermittelt, ob die Okklusion vorliegt. Der Zeitraum von T3 bis T4 wird bei dieser Ausführungsform der Steuerungsvorrichtung von der Auswerteeinheit 11 dazu genutzt, um für diesen Zeitraum von T3 bis T4 das Integral des Quotienten aus dem Istwert des Fluiddurchflusses Q_{IST} und dem Sollwert des Fluiddurchflusses Q_{SOLL} zu berechnen, so dass ein Flächeninhalt A1 bestimmt wird, siehe Fig. 3. Durch vorherige Versuche ist eine Abhängigkeit zwischen einem Integral des Quotienten aus dem Istwert des Fluiddurchflusses Q_{IST} und dem Sollwert des Fluiddurchflusses Q_{SOLL} einerseits und einer Linsenhärte andererseits bestimmt worden. Diese Abhängigkeit kann in einer Tabelle hinterlegt sein, auf welche die Auswerteeinheit 11 zugreifen kann. Somit lässt sich durch die Auswerteeinheit 11 aus dem Integral von Q_{IST} zu Q_{SOLL} bzw. dem bestimmten Flächeninhalt A1 ermitteln, dass eine Härte H1 vorliegt, siehe im Diagramm 35 der Kurvenverlauf 36. Aufgrund dieser Härte H1 wird von der Auswerteeinheit 11 ein erster Betrag E1 einer Ultraschallenergie bestimmt, siehe Diagramm 38 und dort der Kurvenverlauf 39. Mittels einer Steuereinheit 12 kann dann eine Energiequelle 13 so angesteuert werden, dass der erste Betrag E1 einer Ultraschallenergie an das Handstück 3 abgegeben wird.

Wenn ab dem Zeitpunkt T3 bis zum Zeitpunkt T4 der Quotient aus dem Istwert eines Fluiddurchflusses Q_{IST} zu einem Sollwert eines Fluiddurchflusses Q_{SOLL} jedoch auf einen relativ niedrigen Wert absinkt, siehe im Diagramm 31 die Kurve 33, nimmt das Integral des Quotienten über die Zeit einen niedrigeren Betrag an, welcher der Fläche A2 entspricht. Von der Okklusionsbestimmungsvorrichtung 10 wird zwar genauso erkannt, dass eine Okklusion vorliegt. Jedoch wird von der Auswerteeinheit 11 auf der Basis der Relation aus dem Quotienten Q_{IST} zu Q_{SOLL} einerseits und der Linsenhärte andererseits eine Härte H2 ermittelt, welche niedriger ist als die Härte H1, siehe Diagramm 35 und dort Kurvenverlauf 37. Dies bedeutet, dass von der Auswerteeinheit 11 ein erster Betrag E2 einer Ultraschallenergie ermittelt wird, welcher niedriger ist als der erste Betrag E1 bei einem harten Linsenpartikel. Dies ist im Diagramm 38 mit einem Kurvenverlauf 40 dargestellt. Die Auswerteeinheit 11 ist mit einer Steuereinheit 12 so gekoppelt, dass die Steuereinheit 12 während der Okklusion eine Energiequelle 13 so ansteuert, dass die Energiequelle 13 den ermittelten ersten Betrag E2 an die Nadel 2 des Handstückes 3 abgibt.

Wenn der Quotient aus dem Istwert eines Fluiddurchflusses Q_{IST} zum Sollwert des Fluiddurchflusses Q_{SOLL} einen Betrag annimmt, welcher während des Zeitraumes von T2 bis T3 bestand, siehe Diagramm 31, so wird gemäß der ersten Ausführungsform dieser Zeitpunkt als T5 definiert. Gemäß einer zweiten Ausführungsform kann der Zeitpunkt T5 aber als der Zeitpunkt defniert werden, an dem der Quotient aus Q_{IST} zu Q_{SOLL} den Betrag annimmt, der oberhalb des Schwellwertes ist. Zu diesem Zeitpunkt T5 liegt keine Okklusion mehr vor, welches von der Okklusionsbestimmungsvorrichtung 10 erkannt wird. Das Signal der Okklusionsbestimmungsvorrichtung 10 fällt damit von 1 auf 0 zurück, siehe Diagramm 34. Ab diesem Zeitpunkt T5 fehlt die Basis für die Ermittlung einer Härte eines Partikels, so dass die Energiequelle so angesteuert wird, dass ein zweiter Betrag E3 einer Ultraschallenergie abgegeben wird, siehe Diagramm 38. Der zweite Betrag E3 einer Ultraschallenergie kann entweder null sein oder einen minimalen Betrag besitzen. Der Betrag E3 ist jedoch so niedrig, dass eine Emulsifikation nicht möglich ist. Vorzugsweise ist dieser Betrag gerade so hoch, dass Partikel durch die geringe Schwingung der Nadel gemäß dem Prinzip einer Schwingrinne unterstützt werden, durch die Aspirationsleitung zu wandern.

Wenn keine Okklusion bestimmt wird, wird nur ein minimaler oder kein Betrag an Energie zum Handstück zugeführt. Falls eine Okklusion bestimmt wird, wird ein vorbestimmter höherer Betrag an Ultraschallenergie dem Handstück zugeführt, wobei dieser Wert auf der ermittelten Härte des zu zertrümmernden Partikels basiert.

## Patentansprüche

1. Steuerungsvorrichtung (101) für ein ophthalmochirurgisches System (100), aufweisend:
- eine Durchflussbestimmungsvorrichtung (9), mit welcher sich ein Istwert eines Fluiddurchflusses (Q_{IST}) in einer Aspirationsleitung (7) bestimmen lässt, wobei die Aspirationsleitung (7) mit einem Handstück (3) zur Phakoemulsifikation einer Augenlinse (1) gekoppelt ist,
- eine Okklusionsbestimmungsvorrichtung (10), mit welcher sich bestimmen lässt, ob an einer Ansaugöffnung der Aspirationsleitung (7) eine Okklusion durch ein Partikel (21, 22) der Augenlinse (1) vorliegt,
- eine Auswerteeinheit (11), welche geeignet ist, in Abhängigkeit von dem bestimmten Istwert des Fluiddurchflusses (Q_{IST}) in der Aspirationsleitung (7) eine Härte (H1, H2) des Partikels (21, 22) der Augenlinse (1) zu ermitteln, wenn von der Okklusionsbestimmungsvorrichtung (10) bestimmt worden ist, dass eine Okklusion vorliegt, und in Abhängigkeit von der Härte (H1, H2) einen ersten Betrag (E1, E2) einer Ultraschallenergie zu bestimmen, welche einem Handstück (3) mittels einer Energiequelle (13) zuführbar ist, und
- eine Steuereinheit (12), mit welcher sich die Energiequelle (13) so ansteuern lässt, dass sie während der Okklusion den ermittelten ersten Betrag (E1, E2) der Ultraschallenergie abgibt.

2. Steuerungsvorrichtung (101) nach Anspruch 1, wobei die Steuereinheit (12) geeignet ist, die Energiequelle (13) so anzusteuern, dass sie einen zweiten Betrag (E3) einer Ultraschallenergie abgibt, wenn von der Okklusionsbestimmungsvorrichtung (10) bestimmt worden ist, dass keine Okklusion vorliegt, wobei der zweite Betrag (E3) niedriger als der erste Betrag (E1, E2) ist.

3. Steuerungsvorrichtung (101) nach einem der vorherigen Ansprüche, wobei die Okklusionsbestimmungsvorrichtung (10) eine Messvorrichtung zum Messen des von einer Aspirationspumpe (6) aufgenommenen Stroms, oder einer Pumpenleistung, oder des Fluiddurchflusses in der Aspirationsleitung (7) aufweist.

4. Steuerungsvorrichtung (101) nach einem der vorhergehenden Ansprüche, wobei die Auswerteeinheit (11) geeignet ist, die Härte (H1, H2) des Linsenpartikels (21, 22) in Abhängigkeit von einem Quotienten aus dem Istwert des Fluiddurchflusses zu einem Sollwert des Fluiddurchflusses zu ermitteln.

5. Steuerungsvorrichtung (101) nach Anspruch 4, wobei die Auswerteeinheit (11) geeignet ist, während einer vorbestimmten Zeitdauer ein Integral des Quotienten aus dem Istwert des Fluiddurchflusses zu dem Sollwert des Fluiddurchflusses über die Zeit zu bestimmen.

6. Ophthalmochirurgisches System (100) mit einer Steuerungsvorrichtung (101) nach einem der vorherigen Ansprüche.

## Claims

1. Control device (101) for an ophthalmic surgical system (100), comprising:
- a flow determination device (9), by means of which an actual value of a fluid flow (Q_{IST}) in an aspiration line (7) can be determined, wherein the aspiration line (7) is coupled to a handpiece (3) for phacoemulsification of an eye lens (1),
- an occlusion determination device (10), by means of which it is possible to determine whether there is an occlusion as a result of a particle (21, 22) of the eye lens (1) at a suction opening of the aspiration line (7),
- an evaluation unit (11), which is suitable for establishing a hardness (H1, H2) of the particle (21, 22) of the eye lens (1) dependent on the determined actual value of the fluid flow (Q_{IST}) in the aspiration line (7) should the occlusion determination device (10) have determined that an occlusion is present, and, dependent on the hardness (H1, H2), for determining a first quantity (E1, E2) of ultrasound energy, which can be supplied to a handpiece (3) by means of an energy source (13), and
- a control unit (12), by means of which the energy source (13) can be actuated in such a way that, during the occlusion, it emits the established first quantity (E1, E2) of the ultrasound energy.

2. Control device (101) as claimed in Claim 1, wherein the control unit (12) is suitable for actuating the energy source (13) in such a way that it emits a second quantity (E3) of ultrasound energy should the occlusion determination device (10) have determined that there is no occlusion present, wherein the second quantity (E3) is less than the first quantity (E1, E2).

3. Control device (101) as claimed in one of the preceding claims, wherein the occlusion determination device (10) has a measuring device for measuring the current used by an aspiration pump (6), or a pump power or the fluid flow in the aspiration line (7).

4. Control device (101) as claimed in one of the preceding claims, wherein the evaluation unit (11) is suitable for establishing the hardness (H1, H2) of the lens particle (21, 22) depending on a quotient from dividing the actual value of the fluid flow by an intended value of the fluid flow.

5. Control device (101) as claimed in Claim 4, wherein the evaluation unit (11) is suitable for determining, over a predetermined period of time, an integral over time of the quotient from dividing the actual value of the fluid flow by the intended value of the fluid flow.

6. Ophthalmic surgical system (100) with a control device (101) as claimed in one of the preceding claims.

## Revendications

1. Dispositif de commande (101) pour système ophtalmo-chirurgical (100), comprenant :
- un dispositif de détermination de débit (9), au moyen duquel une valeur effective d'un débit de fluide (Q_{IST}) peut être déterminée dans un conduit d'aspiration (7), dans lequel le conduit d'aspiration (7) est raccordé à une pièce à main (3) destiné à la phacoémulsification du cristallin d'un oeil (1),
- un dispositif de détermination d'occlusion (10), permettant de déterminer si une occlusion produite par une particule (21, 22) du cristallin de l'oeil (1) est présente au niveau d'un orifice d'aspiration du conduit d'aspiration (7),
- une unité d'évaluation (11) apte à établir, en fonction de la valeur effective déterminée du débit de fluide (Q_{IST}) dans le conduit d'aspiration (7), une dureté (H1, H2) de la particule (21, 22) du cristallin de l'oeil (1) lorsqu'il a été déterminé par le dispositif de détermination d'occlusion (10) qu'une occlusion était présente, et à déterminer en fonction de la dureté (H1, H2) une première valeur (E1, E2) d'une énergie ultrasonore pouvant être appliquée au moyen d'une source d'énergie (13) à une pièce à main (3), et
- une unité de commande (12), permettant de commander la source d'énergie (13) de manière à délivrer la première valeur (E1, E2) déterminée de l'énergie ultrasonore pendant l'occlusion.

2. Dispositif de commande (101) selon la revendication 1, dans lequel l'unité de commande (12) est apte à commander la source d'énergie (13) de manière à délivrer une seconde valeur (E3) d'une énergie ultrasonore lorsqu'il a été déterminé par le dispositif de détermination d'occlusion (10) qu'une occlusion était présente, dans lequel la seconde valeur (E3) est inférieure à la première valeur (E1, E2).

3. Dispositif de commande (101) selon l'une quelconque des revendications précédentes, dans lequel le dispositif de détermination d'occlusion (10) comprend un dispositif de mesure destiné à mesurer le courant consommé par une pompe d'aspiration (6), ou une puissance de pompe, ou le débit de fluide dans le conduit d'aspiration (7).

4. Dispositif de commande (101) selon l'une quelconque des revendications précédentes, dans lequel l'unité d'évaluation (11) est apte à établir la dureté (H1, H2) de la particule de cristallin (21, 22) en fonction d'un quotient de la valeur effective du débit de fluide à une valeur nominale du débit de fluide.

5. Dispositif de commande (101) selon la revendication 4, dans lequel l'unité d'évaluation (11) est apte à déterminer, pendant une durée prédéterminée, une intégrale au cours du temps du quotient de la valeur effective du débit de fluide à la valeur nominale du débit de fluide.

6. Système ophtalmo-chirurgical (100) comportant un dispositif de commande (101) selon l'une quelconque des revendications précédentes.
